# EUROPEAN PATENT APPLICATION

(11) **EP 4 361 179 A1**
(43) Date of publication of application: **01.05.2024**
(21) Application number: 22828504.5
(22) Date of filing: 23.06.2022
(51) Int. Cl.: C07K 19/00, C12N 15/62, C12N 9/02, C07K 14/415, C07K 14/435

(54) **FUSION PROTEIN, FUSION PROTEIN PRODUCTION METHOD, ELECTRODE, REDOX DEVICE, REDOX METHOD, METHOD FOR CUTTING DISULFIDE BONDS, AND METHOD FOR INACTIVATING ALLERGEN**

(30) Priority: 23.06.2021 JP 2021104343
(71) Applicant: Panasonic Intellectual Property Management Co., Ltd., Osaka-shi, Osaka 571-0057 (JP)
(72) Inventor: HATSUGAI, Noriyuki, Kadoma-shi, Osaka 571-0057 (JP); WAYAMA, Fumiya, Kadoma-shi, Osaka 571-0057 (JP); OKUMURA, Yasuaki, Kadoma-shi, Osaka 571-0057 (JP)
(74) Representative: Novagraaf International SA
(86) International application number: PCT/JP2022/025134
(87) International publication number: WO 2022/270588

(57) **Abstract**

A fusion protein obtained by fusing a ferredoxin-thioredoxin reductase and thioredoxin via a linker peptide. The linker peptide includes glycine (G) and serine (S).

## Description

### [Technical Field]

The present disclosure relates to a fusion protein of a ferredoxin-thioredoxin reductase and thioredoxin and a production method thereof, an electrode on which the fusion protein is immobilized, a redox device including the electrode, a redox method that uses the electrode, a disulfide bond cleavage method, and an allergen inactivation method.

### [Background Art]

In order for thioredoxin to be reduced by the catalytic action of a thioredoxin reductase, thioredoxin and the thioredoxin reductase need to form a heteroprotein complex. For example, Patent Literature (PTL) 1 discloses a method for preparing a protein complex in which thioredoxin and a thioredoxin reductase are fused via an oil body surface-avoiding linker peptide, while retaining activity in association with an oil body in a plant cell.

### [Citation List]

### [Patent Literature]

[PTL 1] Japanese Unexamined Patent Application Publication (Translation of PCT Application) No. 2004-527227

### [Summary of Invention]

### [Technical Problem]

However, in the method disclosed in PTL 1, an oil body surface-avoiding linker peptide located between thioredoxin and a thioredoxin reductase is as long as 49 amino acid residues and further is composed of a negatively charged amino acid sequence, so as to retain activity in association with an oil body in a plant cell. Therefore, the protein complex of thioredoxin and a thioredoxin reductase produced by the method disclosed in PTL 1 has poor flexibility and is not suitable for extracellular use.

In view of this, the present disclosure provides a fusion protein of thioredoxin and a thioredoxin reductase that have been fused via a linker peptide having flexibility and moderate hydrophilicity, and a method of producing the fusion protein.

### [Solution to Problem]

A fusion protein according to an aspect of the present disclosure is a fusion protein obtained by fusing a ferredoxin-thioredoxin reductase and thioredoxin via a linker peptide, wherein the linker peptide includes glycine (G) and serine (S).

### [Advantageous Effects of Invention]

The present disclosure can provide a fusion protein of thioredoxin and a thioredoxin reductase that have been fused via a linker peptide having flexibility and moderate hydrophilicity..

### [Brief Description of Drawings]

[FIG. 1]

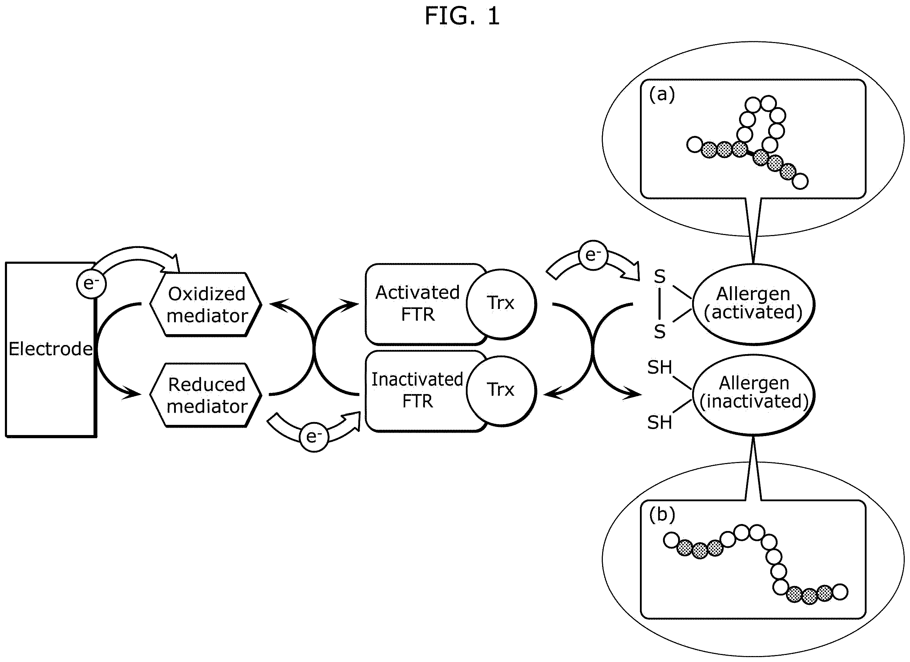

   FIG. 1 is a diagram for describing an application example of a fusion protein according to an embodiment
[FIG. 2]

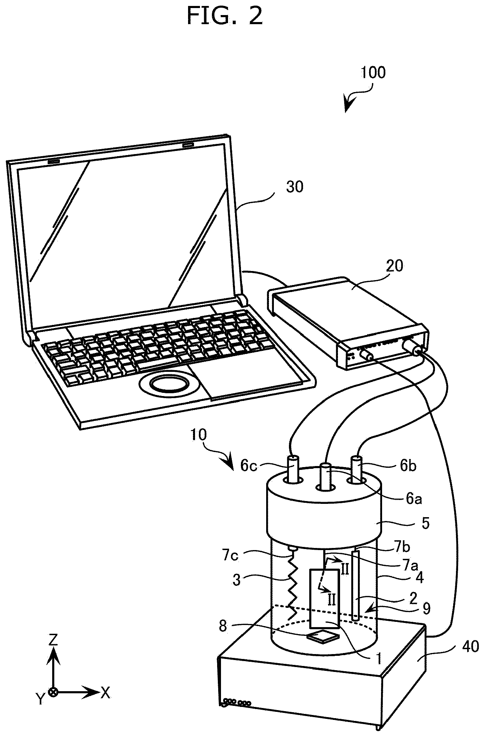

   FIG. 2 is a diagram illustrating an example of a configuration of a redox device including an electrode according to an embodiment.
[FIG. 3]

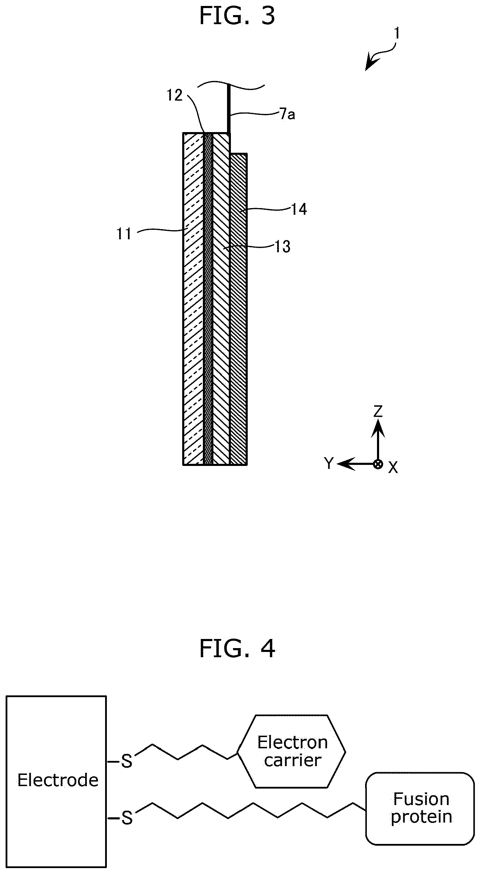

   FIG. 3 is a cross-sectional view taken at line II-II in FIG. 2.
[FIG. 4]
   FIG. 4 is a diagram schematically illustrating an electron carrier and a fusion protein that are immobilized on an electrode.
[FIG. 5]

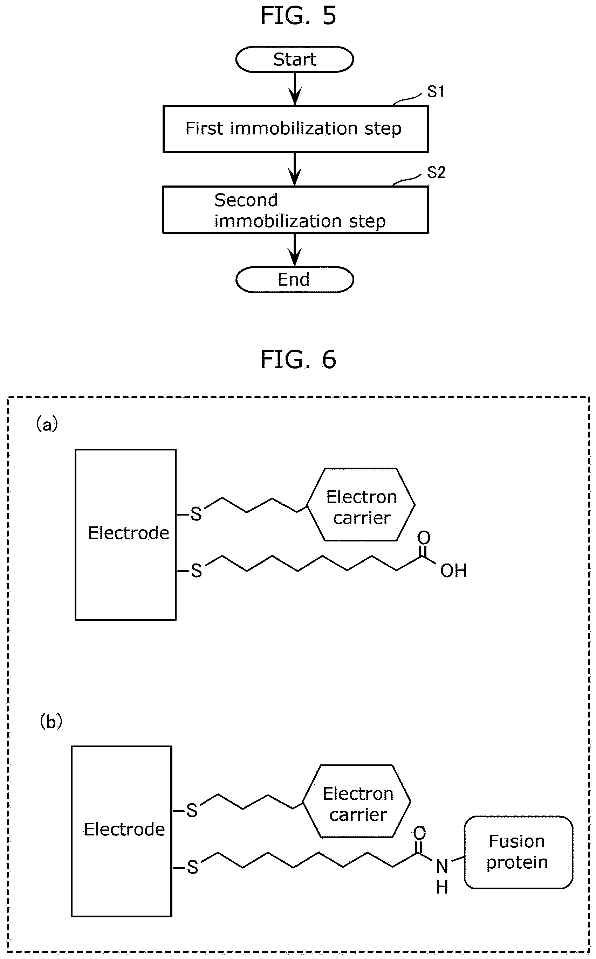

   FIG. 5 is a flowchart illustrating an example of a method of manufacturing an electrode according to an embodiment.
[FIG. 6]
   FIG. 6 is a diagram schematically illustrating respective flows in FIG. 5.
[FIG. 7]

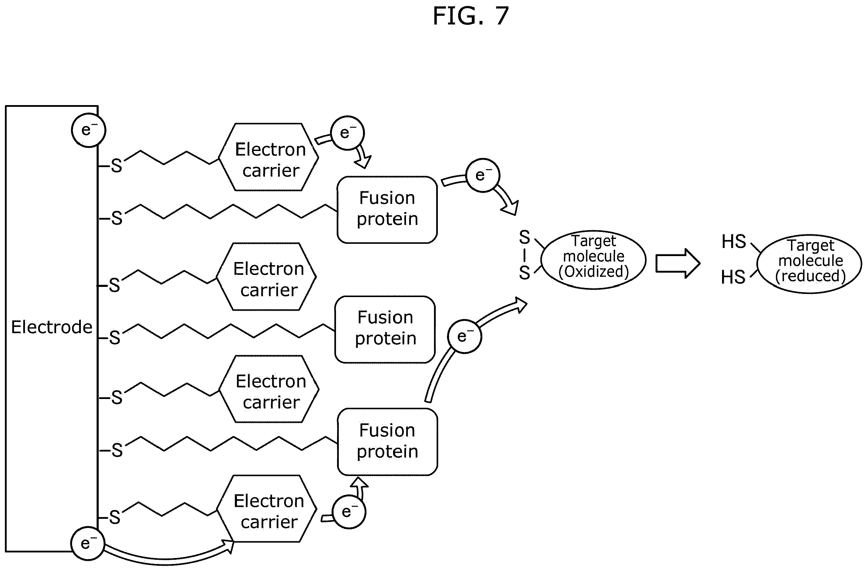

   FIG. 7 is diagram for describing a target molecule redox method.
[FIG. 8]

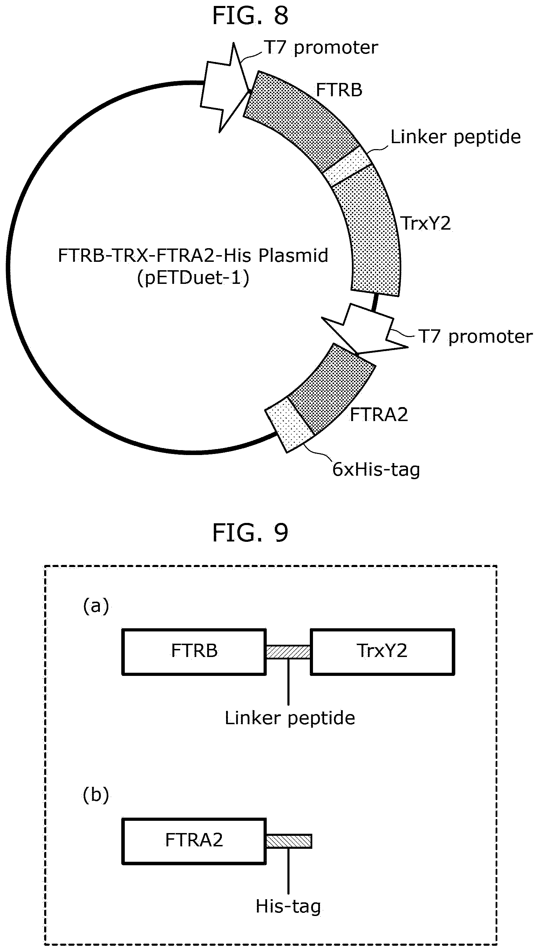

   FIG. 8 is a conceptual diagram of an expression vector for fusion protein production.
[FIG. 9]
   FIG. 9 is a conceptual diagram of fusion proteins.
[FIG. 10]

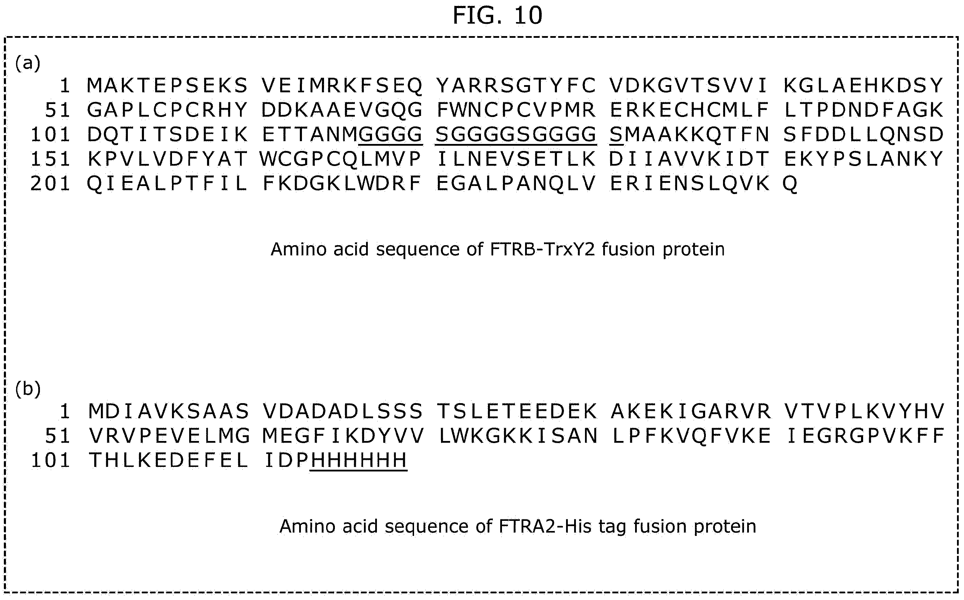

   FIG. 10 is a diagram illustrating amino acid sequences of fusion proteins.
[FIG. 11]

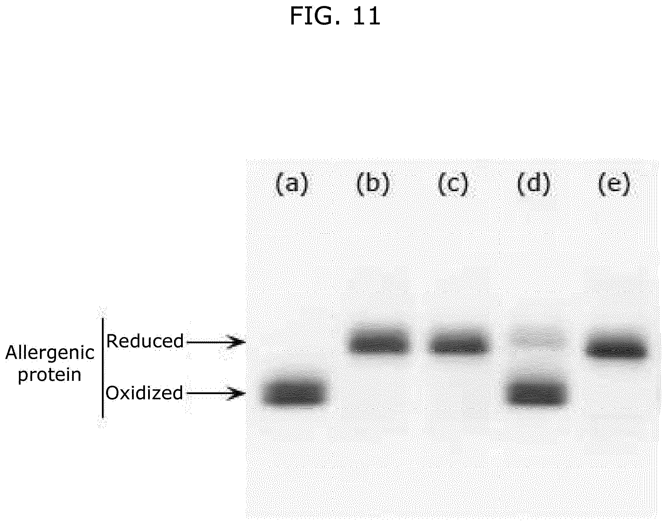

   FIG. 11 is a diagram illustrating SDS-polyacrylamide gel electrophoresis (SDS-PAGE) patterns for evaluation of reductase activity of Comparative Example 1, Comparative Example 2, and Working Example 1.
[FIG. 12]

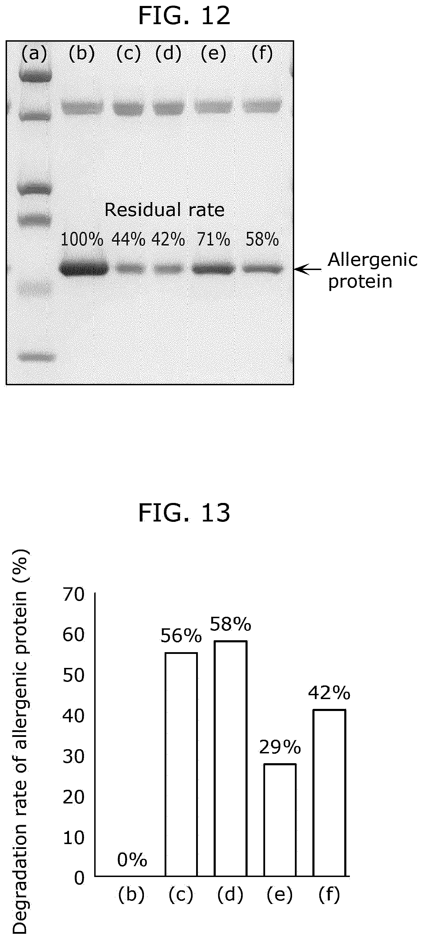

   FIG. 12 is a diagram illustrating electrophoretic patterns after SDS-PAGE for Working Example 3, Working Example 4, Comparative Example 3, and Comparative Example 4.
[FIG. 13]
   FIG. 13 is a graph illustrating calculation results for degradation rates of allergenic proteins.

### [Description of Embodiments]

### (Outline of one aspect of the present disclosure)

An outline of one aspect of the present disclosure is as follows.

A fusion protein according to an aspect of the present disclosure is a fusion protein obtained by fusing a ferredoxin-thioredoxin reductase and thioredoxin via a linker peptide. Here, the linker peptide includes glycine (G) and serine (S).

Accordingly, since the linker peptide includes glycine, which has low molecular weight and a nonpolar side chain, and serine, which has low molecular weight and hydrophilicity, the linker peptide can have flexibility and moderate hydrophilicity.

For example, in the fusion protein according to an aspect of the present disclosure, the linker peptide has an amino acid sequence of more than 5 residues and at most 50 residues.

Accordingly, the proteins fused via the linker peptide can have relative freedom of movement.

For example, in the fusion protein according to an aspect of the present disclosure, the linker peptide: includes at least one glycine and at least one serine; and further includes at least one of (i) H₂N-CHR-COOH where R is either an alkyl group having 1 to 4 carbon atoms or a hydrogen group or (ii) H₂N-CHR-COOH where R is an alkyl group that has 1 or 2 carbon atoms and in which at least one hydrogen group has been substituted with a hydroxyl group.

Accordingly, the linker peptide can maintain flexibility and moderate hydrophilicity even when an amino acid other than glycine and serine and which falls under above-described (i) and (ii) is included.

For example, in the fusion protein according to an aspect of the present disclosure, the linker peptide includes an amino acid sequence of (GGGGS)ₙ, where n is an integer greater than or equal to 2.

Accordingly, proteins to be fused can have relative freedom of movement, and the linker peptide can have flexibility and moderate hydrophilicity.

Furthermore, a fusion protein production method according to an aspect of the present disclosure includes: producing a linker peptide that includes glycine (G) and serine (S); and fusing a ferredoxin-thioredoxin reductase and thioredoxin via the linker peptide produced.

Accordingly, since the fusion protein production method produces a linker peptide that includes glycine, which has low molecular weight and a nonpolar side chain, and serine, which has low molecular weight and hydrophilicity, a fusion protein that is fused via a linker peptide having flexibility and moderate hydrophilicity can be produced.

Furthermore, an electrode according to an aspect of the present disclosure is an electrode on which any one of the above-described fusion proteins is immobilized.

Accordingly, in the electrode, electrodes are transported from the electrode to the fusion protein immobilized on the electrode, and thus electrons can be transported from the fusion protein to a target molecule.

Furthermore, a redox device according to an aspect of the present disclosure includes: the above-described electrode; and a power supply that applies voltage to the electrode. Here, the redox device either oxidizes or reduces a target molecule.

Accordingly, with the redox device, electrons are transported from the electrode to the target molecule, and thus the target molecule can be efficiently oxidized or reduced.

Furthermore, a redox method according to an aspect of the present disclosure includes: oxidizing or reducing a target molecule, using the above-described electrode.

Accordingly, with the redox method, electrons are transported from the electrode to the target molecule, and thus the target molecule can be efficiently oxidized or reduced.

Furthermore, a disulfide bond cleavage method according to an aspect of the present disclosure includes: cleaving a disulfide bond of a protein that is a target molecule, using the above-described electrode.

Accordingly, with the disulfide bond cleavage method, electrons are transported from the electrode to the target molecule, and thus the disulfide bond of the target molecule can be efficiently reduced.

Furthermore, an allergen inactivation method according to an aspect of the present disclosure includes: inactivating an allergen that is a target molecule, using the above-described electrode.

Accordingly, with the allergen inactivation method, electrons are transported from the electrode to the allergen that is the target molecule, and thus the allergen can be efficiently inactivated.

Hereinafter, embodiments will be described with reference to the drawings.

It should be noted that each of the embodiments described shows a general or specific example of the present disclosure. The numerical values, shapes, materials, elements, the arrangement and connection of the elements, steps, the processing order of the steps, etc., indicated in the following embodiments are mere examples, and thus are not intended to limit the claims. Furthermore, among the elements described in the following embodiments, elements not recited in any one of the independent claims that indicate the broadest concepts are described as optional elements. Furthermore, the figures are schematic illustrations and are not necessarily accurate depictions. Elements which are substantially the same have the same reference signs in the figures, and duplicate description may be omitted or simplified.

The mutually orthogonal X-axis, Y-axis, and Z-axis directions illustrated in the figures will be used as appropriate in the description. In particular, the positive side in the Z-axis direction may be described as the upper side, and the negative side in the Z-axis direction may be described as the lower side.

In the present disclosure, terms indicating relationships between elements such as "parallel" and "perpendicular", terms indicating shapes of elements such as "rectangular", and numerical ranges refer not only to their strict meanings, but encompass a range of essentially equivalents, such as a range of deviations of a few percent.

### [Embodiment]

Hereinafter, embodiments will be described with reference to the drawings.

### [1. Fusion protein]

First, the fusion protein according to the present embodiment will be described. The fusion protein according to the present embodiment is a fusion protein obtained by fusing a thioredoxin reductase and thioredoxin via a linker peptide. More specifically, the fusion protein is a fusion protein obtained by fusing an NADPH-thioredoxin reductase or a ferredoxin-thioredoxin reductase (FTR) and thioredoxin (Trx) via a linker peptide, and in other words, is a hybrid enzyme. As illustrated in FIG. 1, the fusion protein donates an electron to a target molecule (here, allergen) upon receiving an electron donated from an electrode to an electron mediator. FIG. 1 is a diagram for describing an application example of the fusion protein according to the present embodiment.

FIG. 1 illustrates an example in which the fusion protein is applied to inactivate an allergen. For example, the allergen is a protein having a disulfide bond, and is also referred to as a so-called allergenic protein. The disulfide bond is a very strong bond that is not easily cleaved by heat, an acid, or an enzyme (for example, gastric digestive enzyme). For this reason, a protein having a disulfide bond is highly likely to cause an allergy. When the fusion protein is applied to inactivate an allergen, the fusion protein donates an electron to the allergen upon accepting an electron donated from the electrode to the electron mediator. Then, the disulfide bond of the allergen (for example, (a) in FIG. 1) is reduced to a thiol group (for example, (b) in FIG. 1) by donating an electron from an activated fusion protein (activated FTR-Trx in FIG. 1). Accordingly, the disulfide bond in the allergenic protein is cleaved to inactivate the allergen.

### [2. Linker peptide]

The linker peptide in the embodiment is a linker peptide for producing a fusion protein by fusing a thioredoxin reductase and thioredoxin. The linker peptide fuses thioredoxin to a thioredoxin reductase such that a redox-active disulfide of thioredoxin can interact with a redox-active disulfide of the thioredoxin reductase.

The linker peptide includes glycine (G) and serine (S). The linker peptide consists of an amino acid sequence of more than 5 residues and 50 residues or less. For example, the linker peptide includes at least one glycine and at least one serine, and further includes at least one of (i) H₂N-CHR-COOH, where R is either an alkyl group having 1 to 4 carbon atoms or a hydrogen group, and (ii) H₂N-CHR-COOH, where R is an alkyl group having 1 or 2 carbon atoms, and at least one hydrogen group of the alkyl group is substituted with a hydroxyl group. For example, the linker peptide includes an amino acid sequence of (GGGGS)ₙ, where n is an integer of 2 or more. In other words, when an amino acid sequence consisting of the 5 residues glycine (G)-glycine (G)-glycine (G)-glycine (G)-serine (S) is taken as one unit, the linker peptide may include 1 or more and 3 or less, preferably 2 or more and 3 or less, or more copies of the amino acid sequence taken as one unit (hereinafter sometimes simply referred to as "GGGGS"). Glycine is a hydrophobic amino acid having no side chain and is more flexible than other amino acids. Serine is a water-soluble amino acid. An investigation for producing a linker peptide having flexibility and moderate hydrophilicity has been performed and as a result has revealed that it is effective to use GGGGS as an amino acid sequence taken as one unit.

On the other hand, PTL 1 discloses a linker peptide consisting of 49 negatively charged amino acid residues, for fusing a thioredoxin reductase and thioredoxin. The linker peptide of the present disclosure consists of an amino acid sequence of 5 to 15 residues including glycine, which has a small side chain, is uncharged, and is more flexible than other amino acids, and serine, which is a water-soluble amino acid. Therefore, the linker peptide of the present invention differs from the linker peptide disclosed in PTL 1 in terms of the configuration and length of the amino acid sequence and also the effects.

### [3. Fusion protein production method]

A fusion protein of a thioredoxin reductase and thioredoxin fused via the above linker peptide can be produced by a conventional technique for fusing the respective proteins.

For example, a DNA encoding a thioredoxin reductase and thioredoxin can be isolated from a plant, an animal, or a microorganism by the polymerase chain reaction (PCR) method. For example, in the case of Arabidopsis thaliana thioredoxin reductase and thioredoxin, the DNA can be isolated by the PCR method from a cDNA after reverse transcription reaction using total RNA extracted from Arabidopsis thaliana. A primer necessary for the isolation can be designed using the sequence information of the gene of each organism. For Arabidopsis thaliana DNA and amino acid sequence information, for example, those registered in the genome database of TAIR (The Arabidopsis Information Resource) or NCBI (National Center for Biotechnology Information) can be utilized.

When producing a fusion protein of a thioredoxin reductase and thioredoxin, a gene encoding the protein can be incorporated into an expression vector according to a known method. The type of the expression vector can be appropriately selected according to the type of a host into which the vector is to be incorporated.

Then, a host such as a microorganism such as Escherichia coli, a plant body, a plant cell, an animal cell, or an insect cell can be transformed with the produced expression vector to express the protein. In addition, the protein may be produced using a cell-free protein expression system without using a host.

### [4. Redox device]

Next, as an application example of the fusion protein according to the present embodiment, a redox device including an electrode on which the fusion protein is immobilized will be described. FIG. 2 is a diagram illustrating an example of a configuration of a redox device including an electrode according to an embodiment.

Redox device 100 includes an electrode on which the fusion protein is immobilized (for example, cathode electrode 1 in FIG. 2) and power supply 20 that applies a voltage to the electrode, and oxidizes or reduces a target molecule. Redox device 100 performs electron transport between the electrode and the fusion protein by applying a voltage to the electrode. Accordingly, electron transfer between the electrode on which the fusion protein is immobilized and a target molecule in sample 9 occurs, and thus the target molecule is oxidized or reduced. For example, redox device 100 applies a voltage to the electrode while a sample 9 (for example, sample solution) containing a target molecule is in a non-flowing state to cause electron transfer between the fusion protein immobilized on the electrode and the target molecule in sample 9, thereby oxidizing or reducing the target molecule. In the example of FIG. 2, redox device 100 includes agitating element 8 and agitator 40 that rotates agitating element 8, but need not agitating element 8 or agitator 40. In the example of FIG. 2, redox device 100 causes agitator 40 to rotate agitating element 8 to switch the sample solution from a non-flowing state to a flowing state, thereby diffusing the oxidized or reduced target molecule into the sample solution. By repeatedly switching the flow state of sample 9 (sample solution) as described above, redox device 100 can efficiently oxidize or reduce the target molecule throughout the entire liquid.

As used herein, a non-flowing state of a solution means, for example, that the solution is not agitated or shaken (for example, not subjected to an external force such as a shearing force or a vibration) and that no fluctuation or other motion is observed on the liquid surface.

Next, the configuration of redox device 100 according to this embodiment will be described with reference to FIG. 2 to FIG. 3.

As illustrated in FIG. 2, redox device 100 includes, for example, agitator 40 that agitates sample 9 containing a target molecule to bring the sample into a flowing state, an electrode (cathode electrode 1) on which a fusion protein that oxidizes or reduces the target molecule by electron transfer with the target molecule is immobilized, power supply 20 that applies a voltage to the electrode, and controller 30 that controls power supply 20 and agitator 40. An electron carrier and the fusion protein are immobilized on the electrode.

Voltage applier 10 is, for example, a three-electrode cell that includes cathode electrode 1 (also referred to as a working electrode), reference electrode 2, counter electrode 3, cell 4, lid 5, terminals 6a, 6b, and 6c, and leads 7a, 7b, and 7c. Voltage applier 10 may be a two-electrode cell that includes, for example, a working electrode (cathode electrode 1) and counter electrode 3.

Cathode electrode 1 and counter electrode 3 are made of a conductive material. The conductive material may be, for example, a carbon material, a conductive polymer material, a semiconductor, or a metal.

First, cathode electrode 1 will be described with reference to FIG. 3 and FIG. 4. FIG. 3 is a cross-sectional view taken at line II-II in FIG. 2. FIG. 4 is a diagram schematically illustrating an electron carrier and a fusion protein that are immobilized on an electrode.

Cathode electrode 1 is an electrode on which a fusion protein is immobilized. Cathode electrode 1 includes, for example, glass substrate 11, titanium deposition layer 12 deposited on glass substrate 11, cathode substrate 13 formed on titanium deposition layer 12, and reaction layer 14 including the electron carrier and the fusion protein that are immobilized on cathode substrate 13.

As cathode substrate 13, for example, a conductive substrate such as gold, glassy carbon, or ITO (Indium Tin Oxide) may be used. The thickness of cathode substrate 13 is not particularly limited.

The electron carrier immobilized on cathode substrate 13 is not particularly limited as long as it is a substance that enables electron transfer between the electrode and the fusion protein immobilized on the electrode. Examples thereof include a viologen, a quinone, or an indophenol. The electron carrier is immobilized on cathode substrate 13 by a first linker in the form of a chain, for example. The first linker includes, for example, an alkyl chain having 2 or more and 5 or less carbon atoms. The first linker has a thiol group at an end thereof. The electron carrier is immobilized on the surface of the electrode by chemical bonding between the first linker and the electrode (specifically, cathode substrate 13).

The electron carrier and the fusion protein immobilized on the electrode may have the following properties (1) to (4). (1) The first linker that connects the electron carrier and the electrode has conductivity, and (2) the second linker that connects the fusion protein and the electrode has a structure similar to that of the first linker but has no conductivity. In addition, (3) the distance between the fusion protein and the electrode is larger than the distance between the electron carrier and the electrode. (4) The distance between the electron carrier and the fusion protein is a distance that allows electron transfer (for example, within several µm). Accordingly, an electron transferred from the electrode to the electron carrier can easily move to the fusion protein. In addition, the fusion protein is disposed on the uppermost surface of the electrode and is immobilized on the electrode by the second linker in the form of a chain, and thus the fusion protein has a higher degree of freedom and more easily acts on the target molecule.

The numbers of carbon atoms of the first linker and the second linker may be changed within the above respective preferable ranges depending on the combination of the electron carrier and the fusion protein.

The second linker having no conductivity includes a case where the second linker conducts electricity slightly but has sufficiently lower conductivity than the first linker and can be regarded as substantially non-conductive.

In addition, the fusion protein immobilized on the surface (so-called cathode substrate 13) of the electrode has a redox protein (for example, thioredoxin) and a redox enzyme (for example, ferredoxin-thioredoxin reductase) fused via a linker peptide, and oxidizes or reduces a target molecule. The fusion protein is immobilized on cathode substrate 13 by the second linker in the form of a chain longer than the first linker. The second linker includes, for example, an alkyl chain having 3 or more and 20 or less carbon atoms, and may include an alkyl chain having 6 or more and 14 or less carbon atoms.

Reference electrode 2 is an electrode that does not react with the components in sample 9 and maintains a constant potential, and is used to control the potential difference between cathode electrode 1 and reference electrode 2 to a constant level by power supply 20. Reference electrode 2 is, for example, a silver/silver chloride electrode. Counter electrode 3 is, for example, a platinum electrode.

Power supply 20 applies a voltage between cathode electrode 1 and counter electrode 3 of voltage applier 10 to control the potential between cathode electrode 1 and reference electrode 2 to a predetermined value in accordance with a control signal output from controller 30.

Controller 30 processes information for controlling the application of a voltage by power supply 20 and the movement of a motor (not illustrated) of agitator 40. Controller 30 is realized, for example, by a processor, a microcomputer, or dedicated circuitry.

Agitator 40 controls the rotation speed and the rotation time of agitating element 8 that is set in voltage applier 10 by controlling the operation of the motor in accordance with a control signal output from controller 30.

### [5. Electrode manufacturing method]

Next, an electrode manufacturing method according to this embodiment will be described with reference to FIG. 5 and FIG. 6. FIG. 5 is a flowchart illustrating an example of an electrode manufacturing method according to this embodiment. FIG. 6 is diagrams schematically illustrating flows in FIG. 5. (a) in FIG. 6 illustrates a first immobilization step, and (b) in FIG. 6 illustrates a second immobilization step. The electrode according to this embodiment is cathode electrode 1.

The electrode manufacturing method includes a first immobilization step (S1) and a second immobilization step (S2) of immobilizing the fusion protein on the electrode via the second linker.

In the first immobilization step (S1), the electron carrier is immobilized on the electrode via the first linker in the form of a chain. First, a solution containing the electron carrier (for example, electron mediator) bonded to the first linker and the second linker is provided, and the first linker bonded to the electron carrier and the second linker are immobilized on the electrode in the same manner as in the formation of a self-assembled monolayer (SAM). For example, the first linker bonded to the electron carrier and the second linker are each mixed into a solution with ethanol:acetonitrile = 1:1, and the electrode is immersed in this mixed solution for 1 hour or more and allowed to stand. As illustrated in (a) in FIG. 6, the electron carrier has the first linker. The first linker has a thiol group at an end thereof. By chemical bonding between the first linker and the electrode, the electron carrier is immobilized on the electrode via the first linker. The first linker is an alkyl chain having a length that allows an electron to move from the electrode to the electron carrier. The first linker is, for example, an alkyl chain having 2 or more and 5 or less carbon atoms.

The second linker for immobilizing the fusion protein to the electrode is an alkyl chain having 3 or more carbon atoms, and may be an alkyl chain having 6 or more carbon atoms in order to prevent an electron from moving from the electrode directly to the fusion protein. In addition, the second linker is an alkyl chain having 20 or less carbon atoms, and may be an alkyl chain having 14 or less carbon atoms in order to allow an electron to easily move from the electron carrier bonded to the first linker to the fusion protein. That is, the second linker has 3 or more and 20 or less carbon atoms, and more preferably, for example, the second linker may be an alkyl chain having 6 or more and 14 or less carbon atoms. In addition, the second linker has a carboxyl group or an amino group at one end thereof and a thiol group at the other end. When a compound having a molecular weight of 500 or less is used as an electron carrier, the second linker may be an alkyl chain having less than 15 carbon atoms. For example, the length of the alkyl chain of the second linker may be appropriately determined according to the length of the alkyl chain of the first linker and the size (molecular weight) of the electron carrier that is bonded to the first linker.

Next, in the second immobilization step (S2), the fusion protein is immobilized on the electrode via the second linker. More specifically, as illustrated in (b) in FIG. 5, the second linker immobilized on the electrode and the fusion protein are bonded to each other. At this time, for example, the amino group of the thioredoxin reductase in the fusion protein and the carboxyl group of the second linker are bonded by an amine coupling reaction. Accordingly, the fusion protein is immobilized on the electrode via the second linker.

### [6. Target molecule redox method]

Next, a target molecule redox method will be described with reference to FIG. 3 and FIG. 7. FIG. 7 is a diagram for describing the target molecule redox method. The target molecule is, for example, a protein or an allergen, and has a disulfide bond.

The target molecule redox method, for example, oxidizes or reduces a target molecule contained in sample 9 by using three-electrode voltage applier 10 including an electrode on which a fusion protein has been immobilized as cathode electrode 1, an anode electrode as counter electrode 3, and reference electrode 2. The surface area of the anode electrode is, for example, sufficiently larger than that of cathode electrode 1.

Sample 9 is an aqueous solution containing a target molecule. The voltage applied to sample 9 may be controlled such that the potential of cathode electrode 1 with respect to reference electrode 2 is the oxidation potential of the electron carrier (electron mediator).

### [Working Examples]

Hereinafter, the present disclosure will be specifically described with reference to Working Examples, but the present disclosure is not limited to only the following Working Examples in any way.

### [Production of fusion protein of a ferredoxin-thioredoxin reductase and thioredoxin]

### [Working Example 1]

In Working Example 1, the configuration of the fusion protein was as follows. In the linker peptide in Working Example 1, an amino acid sequence (GGGGS) consisting of the 5 residues glycine (G)-glycine (G)-glycine (G)-glycine (G)-serine (S) was taken as one unit.

A ferredoxin:thioredoxin reductase (hereinafter, FTR) was used as the thioredoxin reductase, and thioredoxin Y2 (hereinafter, TrxY2) was used as thioredoxin. FTR consists of a catalytic subunit (hereinafter FTRB) and a variable subunit (hereinafter FTRA2), both of which form a heterodimer.

FIG. 8 is a conceptual diagram of an expression vector for fusion protein production. As illustrated in FIG. 8, a gene encoding amino acids 32 to 146 of FTRB (SEQ ID NO: 1) was introduced into the 5' side of multicloning site 1 of an enzyme expression vector (pETDuet-1: produced by Novagen), and a gene encoding amino acids 59 to 167 of TrxY2 (SEQ ID NO: 2) was introduced into the 3' side. Between these two genes, genes encoding linker peptides (SEQ ID NOs: 3 to 5) were introduced to be expressed in required units and in proper order. A gene encoding amino acids 73 to 184 of FTRA2 (SEQ ID NO: 6) and a gene encoding a histidine tag (His-Tag) (SEQ ID NO: 7) were introduced into multicloning site 2.

### Base sequences of inserted genes for expression vector production

(1) A gene encoding amino acids 32 to 146 of FTRB (SEQ ID NO: 1) 
ATGGCGAAAACGGAACCGTCGGAGAAATCAGTAGAGATTATGAGGAA
ATTCTCCGAGCAATATGCTCGTCGCTCTGGGACTTACTTCTGTGTTGA
TAAAGGAGTTACTTCAGTCGTTATTAAGGGTTTGGCTGAGCATAAAGA
TTCATATGGTGCACCGCTTTGCCCTTGCAGACACTATGATGATAAAGC
TGCTGAGGTTGGACAAGGCTTTTGGAATTGTCCGTGTGTTCCAATGA
GAGAGAGGAAGGAGTGCCATTGTATGCTTTTCTTAACTCCTGATAATG
 
ATTTCGCTGGAAAAGATCAGACGATTACATCGGATGAAATAAAAGAAA
CTACAGCTAACATG
(2) A gene encoding amino acids 59 to 167 of TrxY2 (stop codon: TAG) (SEQ ID NO: 2) 
ATGGCAGCAAAGAAGCAAACTTTCAACTCTTTTGATGATTTGTTGCAG
AACTCTGATAAACCTGTACTTGTAGACTTCTATGCTACTTGGTGTGGTC
CATGTCAGCTTATGGTACCTATACTCAATGAAGTGAGTGAAACACTGA
AAGATATAATTGCAGTTGTGAAAATTGATACTGAGAAGTACCCAAGTC
TTGCTAACAAATACCAGATTGAGGCTTTGCCCACATTCATCTTATTCAA
AGACGGCAAACTCTGGGACCGTTTCGAGGGTGCCTTGCCAGCGAAC
CAACTCGTTGAGCGTATTGAGAATTCTTTGCAAGTGAAACAGTAG
(3) Genes encoding linker peptides (SEQ ID NOs: 3 to 5)
   (GGGGS)₁:ggcggcggcggcagc (SEQ ID NO: 3)
   (GGGGS)₂:ggcggcggcggcagcggcggcggcggcagc (SEQ ID NO: 4)
   (GGGGS)₃:ggcggcggcggcagcggcggcggcggcagcggcggcggcggcagc (SEQ ID NO: 5)
(4) A gene encoding amino acids 73 to 184 of FTRA2 (SEQ ID NO: 6) 
ATGGATATTGCTGTGAAATCGGCGGCTTCTGTAGACGCGGACGCTGA
TCTATCGTCATCTACGTCGTTGGAGACGGAGGAAGACGAGAAAGCGA
AGGAGAAGATTGGAGCTAGGGTTAGGGTTACGGTTCCGTTGAAAGTT
TACCATGTGGTTCGTGTACCGGAGGTTGAATTGATGGGAATGGAAGG
TTTTATCAAAGATTACGTCGTTCTTTGGAAAGGAAAGAAAATCTCAGCT
AATCTGCCGTTTAAGGTACAGTTCGTGAAAGAGATCGAAGGTCGTGG
TCCTGTCAAATTCTTCACGCATCTTAAGGAAGACGAGTTCGAGTTAAT
TGATCCG
(5) A gene encoding a histidine tag (His-Tag) (stop codon: TGA) (SEQ ID NO: 7)
   CACCACCACCACCACCACTGA

Next, the produced expression vector was incorporated into Escherichia coli BL21 (DE3) (produced by NIPPON GENE CO., LTD.), and the Escherichia coli was precultured in an LB medium at 37°C overnight. An appropriate amount of the precultured Escherichia coli was inoculated into MagicMedia E. Coli Expression Medium (Invitrogen) and cultured at 16°C overnight. The cells were collected by a centrifugal operation, and a fusion protein was expressed in the cells. FIG. 9 is conceptual diagrams of fusion proteins, and FIG. 10 is diagrams illustrating amino acid sequences. (a) in FIG. 9 is a conceptual diagram illustrating a fusion protein of FTRB and TrxY2, and (b) in FIG. 9 is a conceptual diagram illustrating a fusion protein of FTRA2 and a histidine tag. In addition, (a) in FIG. 10 illustrates the amino acid sequence of the fusion protein of FTRB and TrxY2, and the underlined part illustrates the sequence of a linker peptide. In addition, (b) in FIG. 10 illustrates the amino acid sequence of the fusion protein of FTRA2 and a histidine tag, and the underlined part illustrates the sequence of the histidine tag.

Escherichia coli was suspended in xTractor Buffer (Clontech) and incubated for 10 minutes at 4°C using a rotator. Cell debris was removed by a centrifugal operation at 12,000 × g and 4°C for 20 minutes. The expressed fusion protein was produced from the supernatant after the centrifugal operation using Ni2+ affinity chromatography.

### [Reductase activity evaluation]

### [Working Example 2]

The enzyme activity of the fusion protein produced in Working Example 1 was examined according to the following known method. The reductase activity evaluation method is as follows.

1.0 mM reduced nicotinamide adenine dinucleotide phosphate (NADPH), 0.2 µM ferredoxin:NADPH reductase, 1 µM ferredoxin, and 1 µM of the fusion protein of the present disclosure were mixed into a sample containing an oxidized allergenic protein to prepare an enzyme reaction solution, and the enzyme reaction solution was incubated at 37°C for 1 hour. After that, 10 mM 4-acetamido-4'-maleimidyl-stilbene-2,2'-disulfonate (AMS) was added, and the resulting mixture was incubated at 37°C for 1 hour and then subjected to SDS-PAGE. AMS is a low molecular weight compound that specifically binds to a thiol group, and the number of AMS's that bind differs between a protein that has undergone oxidative modification and a protein that has undergone no oxidative modification, and this difference in number can be detected as a difference in mobility with SDS-PAGE.

FIG. 11 is a diagram illustrating SDS-polyacrylamide gel electrophoresis (SDS-PAGE) patterns for evaluation of reductase activity in Comparative Example 1, Comparative Example 2, and Working Example 1. β-lactoglobulin was used as the allergenic protein.

(b) in FIG. 11 is an electrophoretic pattern when reducing β-lactoglobulin with a fusion protein obtained by fusion via a linker peptide (GGGGS)₃ of 15 amino acid residues shown in SEQ ID NO: 5 of Working Example 1. (c) in FIG. 11 is an electrophoretic pattern when reducing β-lactoglobulin with a fusion protein obtained by fusion via a linker peptide (GGGGS)₂ of 10 amino acid residues shown in SEQ ID NO: 4 of Working Example 1. (d) in FIG. 11 is an electrophoretic pattern when reducing β-lactoglobulin with a fusion protein obtained by fusion via a linker peptide (GGGGS)₁ of 5 amino acid residues shown in SEQ ID NO: 3 of Working Example 1. The reduced form of the allergenic protein was detected in all of (b) to (d) in FIG. 11, confirming reductase activity. It was suggested that among these, the use of a linker peptide of 10 ((c) in FIG. 11) or 15 ((b) in FIG. 11) amino acid residues for the production of a fusion protein provides superior allergenic protein reductase activity.

### [Comparative Example 1]

In Comparative Example 1, the procedure was the same as in Working Example 2, except that a solution obtained by removing the fusion protein from the enzyme reaction solution was used. In this case, as illustrated in (a) in FIG. 11, no reduced form of the allergenic protein was detected, confirming no reductase activity.

### [Comparative Example 2]

In Comparative Example 2, the procedure was the same as in Working Example 2, except that instead of the fusion protein, FTRB and TrxY2 were individually added to the enzyme reaction solution. In this case, as illustrated in (e) in FIG. 11, the reduced form of the allergenic protein was detected, confirming reductase activity.

### [Resolution evaluation of allergenic protein using electrode on which fusion protein is immobilized]

For the reductase activity described above, the fusion protein produced in Working Example 1 was added to the enzyme reaction solution. Hereinafter, the electrode on which the fusion protein produced in Working Example 1 was immobilized was used to evaluate the resolution of an oxidized allergenic protein.

### (1) Preparation of sample containing target molecule

β-lactoglobulin was used as the allergenic protein. A sample containing the allergenic protein (hereinafter referred to as the sample solution) was prepared by dissolving β-lactoglobulin in phosphate-buffered saline (PBS) at pH 7.4 to 3.3 mg/mL.

### (2) Reduction of target molecule using electrode

A predetermined voltage was applied to the sample at a low temperature for 8 hours using a three-electrode voltage application cell (for example, voltage applier 10 in FIG. 2) and a potentiostat (for example, power supply 20 in FIG. 2). An electrode on which the fusion protein produced in Working Example 1 was immobilized was used as cathode electrode 1 of the three electrodes, a Ag/AgCl electrode was used as reference electrode 2 thereof, and a platinum (Pt) electrode was used as counter electrode 3 thereof. The predetermined voltage applied to the sample containing the allergenic protein was controlled by a potentiostat such that the potential of cathode electrode 1 with respect to reference electrode 2 was the reduction potential of the electron mediator. In the electrode, an alkyl chain having 4 carbon atoms was used as the first linker to immobilize an electron carrier (for example, methylviologen) on the electrode, and an alkyl chain having 10 carbon atoms was used as the second linker to immobilize the fusion protein (more specifically, a fusion protein in which a ferredoxin-thioredoxin reductase and thioredoxin are bonded by a linker peptide) on the electrode.

### (3) Confirmation of reduction state of target molecule

In order to confirm that the allergenic protein (here, β-lactoglobulin) had been reduced, the sample after voltage application was reacted with a digestive enzyme at 37°C for 1 hour, and then subjected to SDS-PAGE. Next, the gel after electrophoresis was stained, and the intensity of the allergenic protein band was quantified by image analysis. Electrophoretic patterns are illustrated in FIG. 12. A normal electrode (cathode electrode) on which the fusion protein was not immobilized was used to apply a predetermined voltage to a sample at a low temperature for 8 hours, then the sample was treated with a digestive enzyme, this sample was used as a control sample and subjected to SDS-PAGE, and the resulting numerical value was defined as an allergenic protein residual rate of 100%.

FIG. 12 is a diagram illustrating electrophoretic patterns after SDS-PAGE of Working Example 3, Working Example 4, Comparative Example 3, and Comparative Example 4. FIG. 12 illustrates the allergenic protein residual rates calculated by proportional calculation based on the allergenic protein residual rate of the control sample. (a) in FIG. 12 is an electrophoretic pattern of a molecular weight marker, and (b) in FIG. 12 is an electrophoretic pattern of the control sample. In addition, FIG. 13 is a diagram illustrating the calculation results of the allergenic protein degradation rates. (b) in FIG. 13 is a graph illustrating the allergenic protein degradation rate of the control sample. The allergenic protein degradation rate of the control sample is 0%.

### [Working Example 3]

In Working Example 3, an electrode obtained by immobilizing the fusion protein obtained by fusion via the linker peptide (GGGGS)₃ of 15 amino acid residues shown in SEQ ID NO: 5 of Working Example 1 on the surface of the electrode (cathode electrode 1 in FIG. 2) by the method described in [5. Electrode manufacturing method] was prepared and used. The results are shown in (c) in FIG. 12. (c) in FIG. 12 is an electrophoretic pattern of Working Example 3. In addition, (c) in FIG. 13 is a graph illustrating the allergenic protein degradation rate of Working Example 3. In Working Example 3, it was confirmed that the allergenic protein residual rate was 44%, and that the degradation rate was 56%.

### [Working Example 4]

The procedure was the same as in Working Example 3, except that the fusion protein obtained by fusion via the linker peptide (GGGGS)₂ of 10 amino acid residues shown in SEQ ID NO: 4 of Working Example 1 was used. The results are shown in (d) in FIG. 12. (d) in FIG. 12 is an electrophoretic pattern of Working Example 4. In addition, (d) in FIG. 13 is a graph illustrating the allergenic protein degradation rate of Working Example 4. In Working Example 4, it was confirmed that the allergenic protein residual rate was 42%, and that the degradation rate was 58%.

### [Comparative Example 3]

In Comparative Example 3, the procedure was the same as in Working Example 3, except that the fusion protein obtained by fusion via the linker peptide (GGGGS)₁ of 5 amino acid residues shown in SEQ ID NO: 3 of Working Example 1 was used. The results are shown in (e) in FIG. 12. (e) in FIG. 12 is an electrophoretic pattern of Comparative Example 3. In addition, (e) in FIG. 13 is a graph illustrating the allergenic protein degradation rate of Comparative Example 3. In Comparative Example 3, it was confirmed that the allergenic protein residual rate was 71%, and that the degradation rate was 29%.

### [Comparative Example 4]

In Comparative Example 4, the procedure was the same as in Working Example 3, except that a normal electrode was used and that a ferredoxin-thioredoxin reductase and thioredoxin were individually added to the sample. The results are shown in (f) in FIG. 12. (f) in FIG. 12 is an electrophoretic pattern of Comparative Example 4. In addition, (f) in FIG. 13 is a graph illustrating the allergenic protein degradation rate of Comparative Example 4. In Comparative Example 4, it was confirmed that the allergenic protein residual rate was 58%, and that the degradation rate was 42%.

### (Results)

The results of Working Example 2, Working Example 3, Working Example 4, and Comparative Example 3 (see FIG. 11 and FIG. 12) demonstrate that when the linker peptide in the fusion protein has more than 5 amino acid residues, the allergenic protein degradation rate is higher. It is considered that this is because the degree of freedom of the ferredoxin-thioredoxin reductase and thioredoxin bonded by the linker peptide is increased.

In addition, the results of Working Example 3, Working Example 4, Comparative Example 3, and Comparative Example 4 (see FIG. 12 and FIG. 13) demonstrate that when a fusion protein containing a linker peptide having more than 5 amino acid residues is immobilized on the electrode, the allergenic protein degradation rate is higher than when individually adding a ferredoxin-thioredoxin reductase and thioredoxin to the sample. Therefore, it was confirmed that the disulfide bond of the allergenic protein, which is a target molecule, can be efficiently reduced by using the electrode according to the present disclosure, and thus the allergenic protein can be efficiently degraded.

### (Other embodiments)

Although the immobilized enzyme electrode, the immobilized enzyme electrode manufacturing method, and the target molecule redox method according to the present disclosure have been described above based on embodiments, the present disclosure is not limited to these embodiments. Various modifications to the foregoing embodiments that can be conceived by those skilled in the art as well as other forms resulting from arbitrary combinations of elements in different embodiments that do not materially depart from the essence of the present disclosure are included within the scope of the present disclosure.

### [Industrial Applicability]

The present disclosure is applicable as a fusion protein, a fusion protein production method, an electrode, a redox device, a redox method, a disulfide bond cleavage method, and an allergen inactivation method that are capable of continuously reducing and inactivating allergenic proteins, for example.

### [Reference Signs List]

- 1: cathode electrode
- 2: reference electrode
- 3: counter electrode
- 4: cell
- 5: lid
- 6a: terminal
- 6b: terminal
- 6c: terminal
- 7a: lead
- 7b: lead
- 7c: lead
- 8: agitating element
- 9: sample
- 10: voltage applier
- 11: glass substrate
- 12: titanium deposition layer
- 13: cathode substrate
- 14: reaction layer
- 20: power supply
- 30: controller
- 40: agitator
- 100: redox device

## Claims

1. A fusion protein obtained by fusing a ferredoxin-thioredoxin reductase and thioredoxin via a linker peptide, wherein
the linker peptide includes glycine (G) and serine (S).

2. The fusion protein according to claim 1, wherein
the linker peptide has an amino acid sequence of more than 5 residues and at most 50 residues.

3. The fusion protein according to claim 1 or 2, wherein
the linker peptide:
includes at least one glycine and at least one serine; and
further includes at least one of (i) H₂N-CHR-COOH where R is either an alkyl group having 1 to 4 carbon atoms or a hydrogen group or (ii) H₂N-CHR-COOH where R is an alkyl group that has 1 or 2 carbon atoms and in which at least one hydrogen group has been substituted with a hydroxyl group.

4. The fusion protein according to any one of claims 1 to 3, wherein
the linker peptide includes an amino acid sequence of (GGGGS)ₙ,
where n is an integer greater than or equal to 2.

5. A fusion protein production method comprising:
producing a linker peptide that includes glycine (G) and serine (S); and
fusing a ferredoxin-thioredoxin reductase and thioredoxin via the linker peptide produced.

6. An electrode on which the fusion protein according to any one of claims 1 to 4 is immobilized.

7. A redox device comprising:
the electrode according to claim 6; and
a power supply that applies voltage to the electrode, wherein
the redox device either oxidizes or reduces a target molecule.

8. A redox method comprising:
oxidizing or reducing a target molecule, using the electrode according to claim 6.

9. A disulfide bond cleavage method comprising:
cleaving a disulfide bond of a protein that is a target molecule, using the electrode according to claim 6.

10. An allergen inactivation method comprising:
inactivating an allergen that is a target molecule, using the electrode according to claim 6.
